# EUROPEAN PATENT SPECIFICATION

(11) **EP 3 175 820 B1**
(45) Date of publication and mention of the grant of the patent: **19.03.2025**
(21) Application number: 15814308.1
(22) Date of filing: 01.07.2015
(51) Int. Cl.: A61F 9/007, A61F 2/14

(54) **DRAINAGE DEVICE FOR CONTROLLING INTRAOCULAR PRESSURE IN GLAUCOMA**
DRAINAGEVORRICHTUNG ZUR STEUERUNG DES AUGENINNENDRUCKS BEI GLAUKOM
DISPOSITIF DE DRAINAGE POUR LA RÉGULATION DE LA PRESSION INTRAOCULAIRE D'UN GLAUCOME

(30) Priority: 01.07.2014 PE 00105714
(43) Date of publication of application: 07.06.2017
(73) Proprietor: MM INSTRUMENTS LLC, Wilmington, DE 19808 (US)
(72) Inventor: MIRANDA VELASQUEZ, Mario Eduardo, San Isidro Lima 27 (PE)
(74) Representative: Pons
(86) International application number: PCT/PE2015/000009
(87) International publication number: WO 2016/003294

(56) References cited:
- WO-A1-2004/056294
- US-A- 5 520 631
- US-A- 5 601 094
- US-A- 5 704 907
- US-A- 5 704 907
- US-A- 5 882 327
- US-A1- 2002 156 413
- US-A1- 2004 254 521
- US-A1- 2006 069 340
- US-A1- 2006 155 238
- US-A1- 2006 155 238
- US-A1- 2013 150 770
- US-B2- 7 207 965

## Description

### Technical Field

The present invention generally relates to a surgical treatment for glaucoma and particularly to a one-piece implant allowing the drainage of aqueous humor. This drainage allows controlling pressure within the eye and preventing blindness, pain and other illnesses associated with glaucoma.

### Background of the Invention

Glaucoma is, after diabetes, the second leading cause of blindness in the world. Vision loss as a result of glaucoma involves both central and peripheral vision and has a major impact on the ability of people to live independent lives.

Glaucoma is an optic neuropathy (a disorder of the optic nerve) caused by an elevated intraocular pressure. The high pressure inside the eye compresses the outlet of the optic nerve (papilla) causing changes in its appearance and in the visual function (increase of the optic cup and alterations to the visual field). If the pressure remains increased over a sufficient period of time, a complete and irreversible vision loss takes place.

The intraocular pressure is maintained by a balance between the production and the outflow of aqueous humor. Aqueous humor is a fluid produced by the ciliary body in the posterior chamber of the eye at a rate of approximately 3 to 5 microliters per minute. The fluid produced then passes through the pupillary opening of the iris into the anterior chamber of the eye. Once in the anterior chamber, the fluid leaves the eye through two different routes: the "uveoscleral" route responsible for 10% of the outflow, in which the fluid is filtered between muscle fibers of the ciliary body, and the "canalicular" route responsible for 90% of the outflow , in which the fluid drains through the junction between the cornea and the iris (iridocorneal angle) through a filter called Trabecula which is composed by collagen bundles arranged in a three-dimensional structure similar to a sieve. From that point the aqueous humor goes to the so-called Schlemm's canal and then to the episcleral veins.

As the eye is a closed area, the production and outflow of aqueous humor necessarily have to be equal. This causes a pressure inside the eye that is determined by the amount of fluid produced and the capacity of outflow thereof. In normal conditions, the intraocular pressure is between 10 and 21 mm Hg.

If aqueous humor production increases with respect to outflow resistance, the intraocular pressure will increase to force the outflow. If the outflow resistance increases, the intraocular pressure will increase to force the outflow even with a normal aqueous humor production. Both scenarios will increase intraocular pressure and may cause glaucoma, being much more frequent the increase in the outflow resistance.

Aqueous humor outflow resistance may be increased by several factors. Thus, in the so-called "primary open angle glaucoma" (responsible for 85% of glaucoma), the abnormal resistance is produced along the outer aspect of the trabecular meshwork and the inner wall of the Schlemm's canal. In "angle-closure glaucoma", the resistance increases due to mechanical blocking of the angle and in "secondary glaucoma" due to various reasons such as inflammatory waste deposits, vascularization of the area, pigment blocking, etc.

Regardless of the factor that produces it, increased intraocular pressure compresses the outlet of the optic nerve and interferes in its vascularization, causing the death of nerve cells that carry visual stimuli to the brain, producing alterations in the visual field and, in advanced cases, total and irreversible blindness.

The only therapeutic approach currently available for glaucoma is to reduce intraocular pressure. The clinical treatment of glaucoma is done step by step, the first step being the medical treatment either by oral means or with drops.

The drugs work by reducing aqueous humor production or by facilitating its outflow. Currently available medicinal products can have many side effects, some serious, including congestive heart failure, respiratory distress, hypertension, depression, kidney stones, aplastic anemia, sexual dysfunction and death. Compliance with medication is also a major problem with more than half of glaucoma patients being estimated as not following their dosing schedules correctly.

While drugs work, they will be the treatment of choice, but often they are not sufficient to maintain a safe intraocular pressure and surgery is needed. The most frequently performed surgeries are trabeculoplasty and trabeculectomy.

In laser trabeculoplasty, thermal energy from a laser is applied to a number of noncontiguous spots in the trabecular meshwork. It is believed that the laser energy somehow stimulates the metabolism of the trabecular cells and changes the extracellular material of the trabecular meshwork. In approximately 80% of patients, aqueous outflow is increased and the pressure decreases. However, the effect often is not long lasting and 50% of patients develop hypertension again in five years. In addition, laser trabeculoplasty is not an effective treatment for primary open angle glaucoma in patients of less than fifty years old nor is it effective for angle-closure glaucoma and many secondary glaucomas.

If laser trabeculoplasty does not reduce the pressure to the desired level, filtering surgery is then performed.

The most commonly performed filtering procedure is trabeculectomy. In a trabeculectomy an incision is made in the conjunctiva, which is the transparent tissue that covers the sclera. The conjunctival tissue is lifted and a scleral flap of approximately 4x4 mm is made with a depth of 50% of the scleral thickness and is lamellar dissected up to 1 mm into the cornea. The anterior chamber is entered beneath the scleral flap and a section of the deep sclera and trabecular meshwork is excised. The scleral flap is loosely sewn back into place and conjunctival incision is closed well. Postoperatively, the aqueous humor passes through the hole beneath the scleral flap and goes to the subconjunctival space where it forms a blister from where it passes to the blood vessels of the conjunctiva or goes through the conjunctiva to the surface.

Trabeculectomy is associated with many problems, among them, the fibroblasts that are present in the episclera proliferate, migrate and can scar the scleral flap, failures in healing may occur, particularly in children and young adults. Of the eyes that have had an initially successful trabeculectomy, 80% will stop filtering in a period of 3 to 5 years after surgery. To minimize fibrosis, surgeons are now applying antifibrotic agents such as mitomycin C (MMC) and 5-fluorouracil (5-FU) to the scleral flap at the time of surgery. The use of these agents has increased the success rate of trabeculectomy, but also has increased its complications.

When trabeculectomy does not successfully lower the eye pressure, the next surgical step is often an aqueous shunt valve. This device consists of a silicone tube that is attached at one end to a plastic (polypropylene or other synthetic) plate with a valve shape that is sewn to the ocular equator or an element found thereafter and the other end of the silicone tube is introduced into the anterior chamber through a hole made in the corneal limbus. The outer portion of the tube is covered with sclera or pericardium from donor, it is all covered with the conjunctiva and the incision is closed. The current technology of these aqueous shunt valves presents many problems, including poor healing, valve failure, hypotony and infection.

In the year 1999, the company Optonol Ltds. patented a tubular filtering device not valved for controlling intraocular pressure in glaucoma (Patent 5968058). This device was a small stainless steel tube that, once placed in the corneal limbus, allowed the flow of aqueous humor through the tube from the anterior chamber to an intrascleral space and from there to the subconjunctival space. Since 2002, the company Alcon sells this device under the name of Ex-Press.

The implantation of these devices allows performing less invasive surgeries compared to trabeculectomy as it allows the aqueous humor outflow without the need of extracting a segment of the trabecula or performing an iridectomy.

Several studies have reported the effectiveness of these devices for significantly reducing intraocular pressure, but they also report complications, the most common ones being a flat chamber and bleeding in anterior chamber, which is believed may be related with the anchorage system.

All the surgical techniques to reduce intraocular pressure seek to create a new drainage pathway for aqueous humor that replaces the obstructed normal path with the smallest possible amount of complications.

Patent document WO 2004/056294 discloses an implant that has drainage passageways for draining ocular fluid from the anterior chamber. The passageways are formed in outer surfaces or through the interior of the implant. Interior passageways are formed in surfaces of layers of the implant or by voids in inner layers of the implant. Patent document US 2004/254521 discloses a system for reducing intraocular hypertension, comprising an implantable shunt, comprising a planar member having at least one microchannel disposed within the planar member. There is an inflow port disposed proximate to a first end of the microchannel and an outflow port disposed proximate to a second end of the microchannel. Patent document US 5 520 631 discloses a method and an apparatus for lowering the intraocular pressure of an eye. In the method of the invention, an opening is created in the limbus cornea region of the eye. A filtering implant having a foot portion and a body portion is provided for implantation. The foot portion of the implant is placed through the limbal opening into the anterior chamber of the eye, and the body portion is buried beneath a scleral flap. The apparatus of the invention is a filtering implant. Patent document US 5 704 907 discloses a method and an apparatus for lowering the intraocular pressure of an eye are provided. Patent document The apparatus of the invention is a filtering implant adapted to extend from the anterior chamber of the eye through an opening in the limbus corneae to a drainage area beneath a scleral flap. Patent document US 2006/155238 discloses an ophthalmic shunt implantable in an eye having an elongate body and a conduit for conducting aqueous humor from an anterior chamber of the eye to the suprachoroidal space of the eye. The elongate body has a forward end and an insertion head that extends from the forward end. The insertion head defines a shearing edge suitable for cutting eye tissue engage thereby. The forward end and the insertion head of the body define a shoulder surface. The conduit has a first end defined on a portion of a top surface of the insertion head. The conduit also extends through the body from the forward end to a back end thereof. The first end of the conduit is spaced from the shearing edge and, in one example, from the shoulder of the body. Finally, document US 2006/069340 discloses a system for reducing intraocular pressure, the system having: an implantable shunt, the implantable shunt with a planar member, at least one microchannel disposed within that planar member, and a laser whereby at least one fenestration may be introduced into the microchannel.

### Description of the Invention

The present invention discloses a one-piece implant for drainage of aqueous humor with higher or lower speed depending on the pressure gradient between the interior and the exterior of the eye by pressure of the aqueous humor of the eye according to claim 1.

Particular embodiments of the implant can be found in the dependent claims.

### Brief Description of the Present Invention

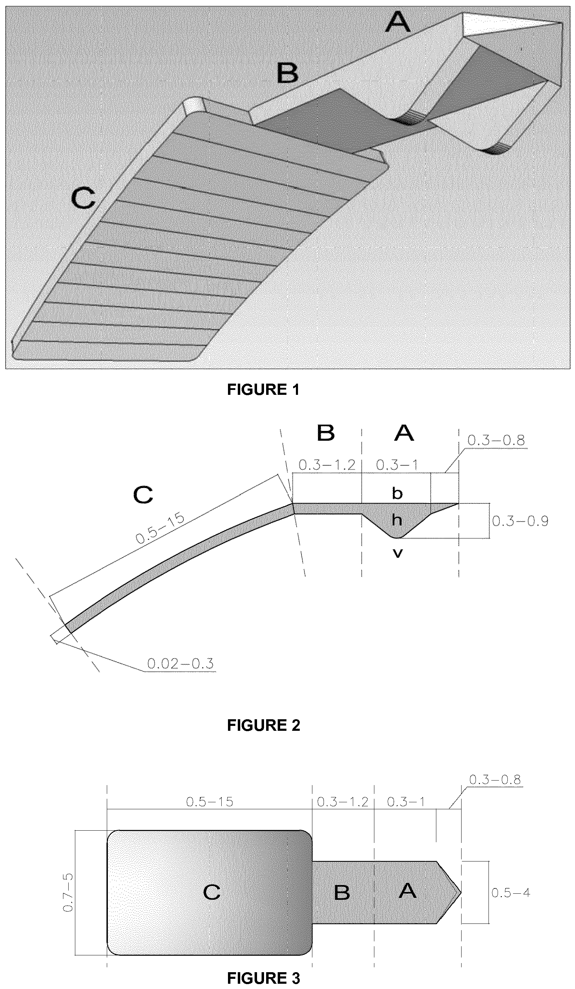

**Figure 1** shows a **three-dimensional view** of the device object of the present invention which has been divided into three parts: "A", "B" and "C". Part "A" will be arranged in the anterior chamber between the cornea and the iris and will have a double triangle shape, which allows introducing and removing it with ease while at the same time keeping it fixed in its position. Part "B" will puncture the eye in the corneal limbus, being arranged between the cornea and the sclera, and part "C" will be arranged outside the eye covered by a scleral flap, on the episclera or both.
**Figure 2** shows **a side view** of the device object of the present invention which has been divided into three parts: "A", "B" and "C". Part "A" will be arranged in the anterior chamber between the cornea and the iris and will have a double triangle shape, which allows introducing and removing it with ease while at the same time keeping it fixed in its position. Part "B" will puncture the eye in the corneal limbus and part "C" will be arranged outside the eye covered by a scleral flap, on the episclera or both.
**Figure 3** shows a **top view** of the device object of the present invention which has been divided into three parts: "A", "B" and "C". Part "A" will be arranged in the anterior chamber between the cornea and the iris, part "B" will puncture the eye in the corneal limbus and part "C" (which can have variable measurements) will be arranged outside the eye.

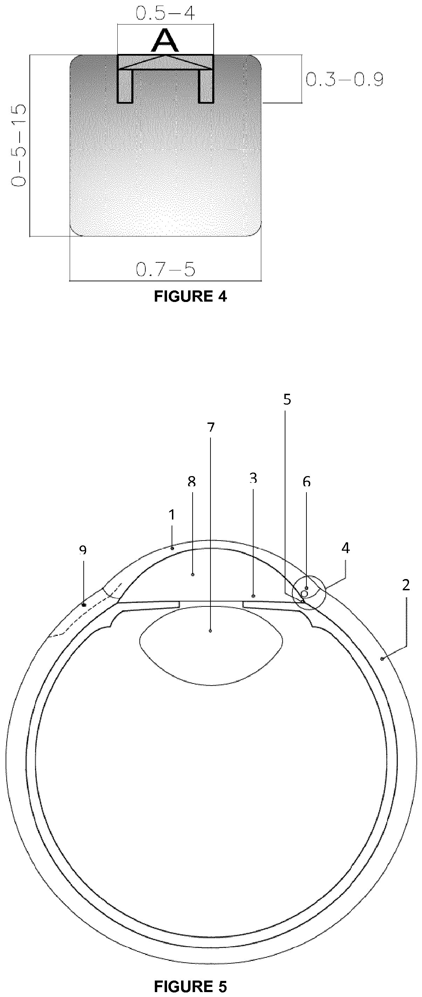

**Figure 4** shows a **front view** of the device object of the present invention which has been divided into three parts: "A", "B" and "C". Part "A" will be arranged in the anterior chamber between the cornea and the iris, part "B" will puncture the eye in the corneal limbus and part "C" (which can have variable measurements) will be arranged outside the eye.
**Figure 5** is an illustration showing a sagittal section diagram of the human eye depicting therein the anatomical details of the iridocorneal angle which is the area on which the present invention will influence. The following can be seen therein: 1. Cornea, 2. Sclera, 3. Iris, 4. Corneal limbus, 5. Trabecula, 6. Schlemm's canal 7. Crystalline lens. The indication 8. Flap shows the area of the eye where the flap or scleral flap will be made. Part "C" of the device will be arranged between the scleral flap and the sclera, creating two virtual spaces, one towards the flap and another towards the sclera. Aqueous humor will flow through these virtual spaces and be diffused into the interscleral space and subconjuntival space.

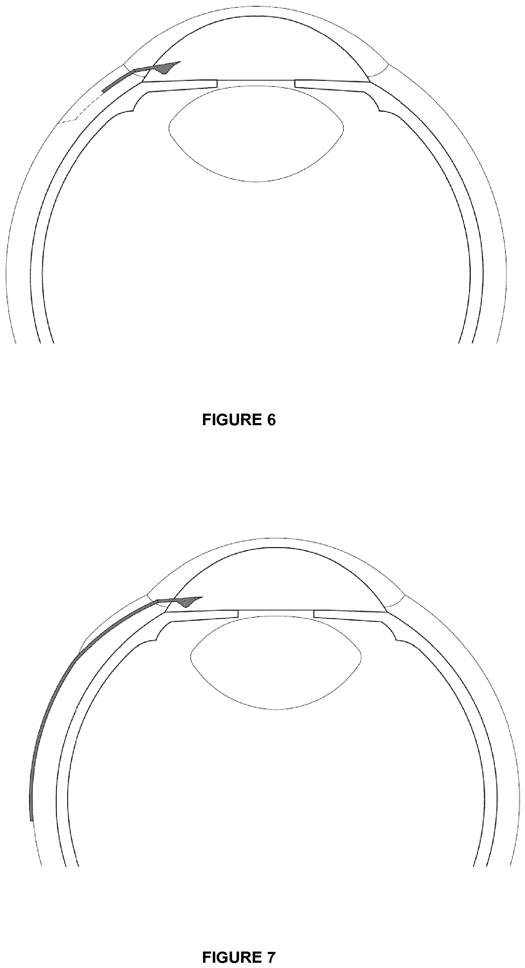

**Figure 6** is an illustration showing the device object of the present invention in its working position and in which: Part "A" will be arranged in the anterior chamber. Part "B" will puncture the eye and create a virtual space upwardly between the cornea and the upper surface of the device and create a virtual space downwardly between the sclera and the lower surface of the device. Part "C" will be arranged outside the eye between the scleral flap and the sclera, similarly creating two virtual spaces, one towards the flap and another towards the sclera. Aqueous humor will flow through these virtual spaces and be diffused into the interscleral space and subconjuntival space.
**Figure 7** is an illustration similar to that of Figure 6 but in which: Part "C" is longer and, in addition to being arranged between the scleral flap and the sclera, it comes out the flap to the episcleral space, creating a virtual corridor that leads to the ocular equator, increasing the area of diffusion of the aqueous humor. This can be beneficial in cases of neovascular glaucoma.

### Detailed Description of the Present Invention

The present invention provides a device for permanent insertion into the scleral angle of the eye to reduce intraocular pressure and control glaucoma.

Since the device is a long-term implant, it must be manufactured with a material that is harmless to the tissues and fluids with which it will be in contact. The device must not be absorbed, corroded or structurally compromised while it is being arranged in place. In addition, it is equally important that the eye tissues and the aqueous humor are not negatively affected by the presence of the implanted device.

The device is made of stainless steel or other biocompatible materials that do not adhere to the eye tissues which may include but are not limited to, among others, silicone or similar polymers, polytetrafluoroethylene, polypropylene or other thin wall polymers. Metals and alloys known in the art for making implants can also used. It is important that the material used does not adhere to the eye tissues in order to create one or more virtual spaces from the anterior chamber to the exterior of the eye, be it an intrascleral space, an episcleral space or both. The space is virtual, not real, which creates a one-direction path through which the aqueous humor will flow with higher or lower speed depending on the pressure gradient between the interior and the exterior of the eye. Since the drainage path is a virtual path, it minimizes the possibility of the chamber being flat or of germs penetrating through the device, furthermore the anchorage system is less aggressive, thus reducing the possibility of bleeding.

**Figure 1** shows a three-dimensional view of the device object of the present invention which has been divided into three parts: "A", "B" and "C". This device will be made of stainless steel or another biocompatible material that does not adhere to the eye tissues, the device will have a uniform or variable thickness. Part "A" will be arranged in the anterior chamber between the cornea and the iris and will have a double triangle shape, which allows introducing and removing it with ease while at the same time keeping it fixed in its position. Part "B" will puncture the eye in the corneal limbus, being arranged between the cornea and the sclera, and part "C" will be arranged outside the eye covered by a scleral flap, on the episclera or both.

**Figure 2** shows a side view of the device object of the present invention. The device will have a uniform or variable thickness and will measure between 0.02 and 0.3 mm. The device is a one-piece device, but it has been divided into in three parts for description purposes: "A", "B" and "C". Part "A" will be arranged inside the eye in the anterior chamber between the cornea and the iris. Part "B" will puncture the corneal limbus forming two virtual spaces through which it will drain the aqueous humor and part "C" will be arranged outside the eye, in the interscleral space, episcleral space or both. The parts forming the device of the invention are:

### Part "A":

**Figure 2** shows a side view of a filtering device in which part "A", which will be arranged in the anterior chamber between the cornea and the iris (see Figure 6), can be seen. This part "A" will have a double triangle shape with an upper base "b" having measurements between 0.3 and 1.0 mm, height "h" between 0.3 and 0.9 mm and a rounded lower vertex "v" to facilitate its insertion. This allows a less traumatic anchorage in addition to facilitating its introduction or removal.

**Figure 3** shows a top view of the device in which part "A" can be seen. This part has a rectangular shape in which it is continued with part "B" on the proximal side and becomes a triangle on the distal side. The rectangular part will have a width between 0.5 and 4 mm equal to that of part B and a length between 0.3 and 1.2 mm. The triangular distal part will have a base of the same width as the rectangular part and a height between 0.3 and 0.8 mm. The edges will be sharp to facilitate insertion.

### Part "B":

**Figure 2** shows a side view of the filtering device in which part "B" can be seen. This part will puncture the corneal limbus, forming a virtual space upwardly between the cornea and the upper surface of the device and another virtual space downwardly between the sclera and the lower surface of the device. The aqueous humor will drain through these virtual spaces from the anterior chamber to an intrascleral or episcleral space. The sheet forming part "B" of the device will have a thickness between 0.02 and 0.3 mm.

Figure 3 shows a top view of the device in which part "B" can be seen, the upper surface of which will be in contact with the cornea and form with it a virtual space through which the aqueous humor will flow. The lower surface will be in contact with the sclera and form another virtual space. The surfaces of this sheet can be smooth, rough, wavy, grooved or with another finish. Part "B" of the device will have a length between 0.3 and 1.2 mm with a width equal to that of part "A", this width will measure between 0.5 and 4 mm.

### Part "C"

**Figure 2** shows a side view of the filtering device in which part "C" can be seen. This part will have a length between 0.5 and 15 mm and a thickness between 0.02 and 0.3 mm, it will be thin, malleable and will be curved to approximately the eye curvature.

**Figure 3** shows a top view of the device in which part "C", being in the form of a rectangular sheet with rounded edges and wider than part "B", can be seen. It will measure between 0.7 and 5 mm in width and will have a length between 0.5 and 15 mm. This sheet can be smooth, rough, wavy, grooved or with another finish, it will be arranged outside the eye and covered by a scleral flap, arranged in the episcleral space or both.

**Figure 5** is an illustration showing the area of the eye where the flap or scleral flap will be made to create the interscleral virtual space. This flap will be similar to that obtained for a trabeculectomy surgery and will have a length of approximately 4 mm, a width of 4 mm and a depth of about 50% the scleral thickness.

**Figure 6** is an illustration showing the device object of the present invention in its working position and in which part "A" will be arranged in the anterior chamber. Part "B" will puncture the eye and create a virtual space upwardly between the cornea and the upper surface of the device and create a virtual space downwardly between the sclera and the lower surface of the device. Part "C" will be arranged outside the eye between the scleral flap and the sclera, similarly creating two virtual spaces, one towards the flap and another towards the sclera. Aqueous humor will flow through these virtual spaces and be diffused into the interscleral space and subconjuntival space.

**Figure 7** is an illustration similar to that of **Figure 6** but in which part "C" is longer and, in addition to being arranged between the scleral flap and the sclera, it comes out the flap towards the episcleral space, creating a virtual corridor that leads to the ocular equator, increasing the area of diffusion of the aqueous humor. This can be very beneficial in cases of neovascular glaucoma.

The surgical procedure needed to insert the device can include all or some of the following steps: **1.** an incision is made in the conjunctiva that may be limbus or fornix based, **2.** a scleral flap of approximately 4 x 4 mm with a thickness of approximately 50% the scleral thickness is delimited and a dissection parallel to the sclera is made until entering 1 mm into the corneal tissue, **3.** paracentesis is performed parallel to the limbus with a blade of 15°,**4.** the device is inserted into the paracentesis, **5.** the flap is closed with 2 loose stitches, **6.** the conjunctiva is closed.

## Claims

1. One-piece implant for drainage of aqueous humor with higher or lower speed depending on the pressure gradient between the interior and the exterior of the eye, the implant being made of a material which does not adhere to eye tissue and comprising three parts:
a first part (A) suitable to be located in the anterior chamber of the eye,
a second part (B) that has a sheet form and is suitable to puncture the corneal limbus to be arranged between the cornea and the sclera, and
a third part (C) having a sheet form suitable to be located outside of the eye,
wherein the implant is made of stainless steel, silicone, polytetrafluoroethylene, polypropylene, or a biocompatible metal or alloy;
wherein the second part (B) forms a space upwardly between the cornea and the upper surface of the implant, and a space downwardly between the sclera and the lower surface of the implant wherein these spaces create a one direction path through which the aqueous humor will flow depending on the pressure gradient between the interior and the exterior of the eye;
**characterised in that** the first part (A) is provided with an upper base and a double triangle shape protruding in a direction perpendicular to the upper base which allows for introducing and removing the implant with ease while at the same time keeping the implant fixed in position.

2. The implant of claim 1 wherein the double triangle shape has a rounded lower vertex (v).

3. The implant of claim 1 wherein the upper base has a rectangular shape, wherein a distal side of the rectangular shape continues and becomes a triangle provided with sharp edges.

4. The implant of claim 3 wherein the second part (B) is rectangular and has the same width as the upper base.

5. The implant of claim 1 wherein the third part (C) has a rectangular shape with rounded corners.

6. The implant of claim 4, wherein the first part (A) has a width between 0.5 and 4 mm.

7. The implant of claim 4, wherein the second part (B) has a length between 0.3 and 1.2 mm; a width between 0.5 and 4 mm; and a thickness between 0.02 and 0.3 mm.

8. The implant of claim 1 wherein the third part (C) has a length between 0.5 and 15 mm, a width between 0.7 and 5 mm and a thickness between 0.02 and 0.3 mm.

## Patentansprüche

1. Einteiliges Implantat zur Drainage des Kammerwassers mit höherer oder niedrigerer Geschwindigkeit je nach Druckgefälle zwischen dem Inneren und dem Äußeren des Auges,
wobei das Implantat aus einem Material hergestellt ist, das nicht am Augengewebe haftet, und drei Teile umfasst:
einen ersten Teil (A), der geeignet ist, um in der Vorderkammer des Auges angebracht zu werden,
einen zweiten Teil (B), der eine Blattform aufweist und geeignet ist, den Limbus corneae zu durchstechen, um zwischen der Hornhaut und der Sklera angeordnet zu werden, und
einen dritten Teil (C), der eine Blattform aufweist und geeignet ist, außerhalb des Auges angebracht zu werden,
wobei das Implantat aus rostfreiem Stahl, Silikon, Polytetrafluorethylen, Polypropylen oder einem biokompatiblen Metall oder einer biokompatiblen Legierung hergestellt ist;
wobei der zweite Teil (B) einen Raum nach oben zwischen der Hornhaut und der oberen Fläche des Implantats und einen Raum nach unten zwischen der Sklera und der unteren Fläche des Implantats bildet,
wobei diese Räume einen Weg in eine Richtung bilden, durch den das Kammerwasser je nach Druckgefälle zwischen dem Inneren und dem Äußeren des Auges fließt;
**dadurch gekennzeichnet, dass** der erste Teil (A) mit einer oberen Basis und einer doppelten Dreiecksform, die in einer Richtung senkrecht zur oberen Basis hervorsteht, versehen ist, was ein einfaches Einführen und Entfernen des Implantats ermöglicht, während das Implantat gleichzeitig an Ort und Stelle fixiert bleibt.

2. Implantat nach Anspruch 1, wobei die doppelte Dreiecksform einen abgerundeten unteren Scheitelpunkt (v) aufweist.

3. Implantat nach Anspruch 1, wobei die obere Basis eine rechteckige Form aufweist, wobei sich eine distale Seite der rechteckigen Form fortsetzt und in ein mit scharfen Kanten versehenes Dreieck übergeht.

4. Implantat nach Anspruch 3, wobei der zweite Teil (B) rechteckig ist und die gleiche Breite wie die obere Basis aufweist.

5. Implantat nach Anspruch 1, wobei der dritte Teil (C) eine rechteckige Form mit abgerundeten Ecken aufweist.

6. Implantat nach Anspruch 4, wobei der erste Teil (A) eine Breite zwischen 0,5 und 4 mm aufweist.

7. Implantat nach Anspruch 4, wobei der zweite Teil (B) eine Länge zwischen 0,3 und 1,2 mm; eine Breite zwischen 0,5 und 4 mm; und eine Dicke zwischen 0,02 und 0,3 mm aufweist.

8. Implantat nach Anspruch 1, wobei der dritte Teil (C) eine Länge zwischen 0,5 und 15 mm, eine Breite zwischen 0,7 und 5 mm und eine Dicke zwischen 0,02 und 0,3 mm aufweist.

## Revendications

1. Implant monobloc pour le drainage de l'humeur aqueuse avec une vitesse plus ou moins élevée en fonction du gradient de pression entre l'intérieur et l'extérieur de l'œil,
l'implant étant constitué d'un matériau qui n'adhère pas au tissu oculaire et comprenant trois parties :
une première partie (A) appropriée pour être placée dans la chambre antérieure de l'oeil,
une deuxième partie (B) qui a une forme de feuille et qui est appropriée pour perforer le limbe cornéen pour être agencée entre la cornée et la sclérotique, et
une troisième partie (C) ayant une forme de feuille appropriée pour être placée à l'extérieur de l'œil,
dans lequel l'implant est constitué d'acier inoxydable, de silicone, de polytétrafluoroéthylène, de polypropylène, ou d'un métal ou d'un alliage biocompatible ;
dans lequel la deuxième partie (B) forme un espace vers le haut entre la cornée et la surface supérieure de l'implant, et un espace vers le bas entre la sclérotique et la surface inférieure de l'implant
dans lequel ces espaces créent une voie unidirectionnelle par laquelle l'humeur aqueuse s'écoulera en fonction du gradient de pression entre l'intérieur et l'extérieur de l'œil ;
**caractérisé en ce que** la première partie (A) est pourvue d'une base supérieure et d'une forme de double triangle faisant saillie dans une direction perpendiculaire à la base supérieure, ce qui permet d'introduire et de retirer facilement l'implant tout en le maintenant en même temps fixé en position.

2. Implant de la revendication 1 dans lequel la forme de double triangle a un sommet inférieur arrondi (v).

3. Implant de la revendication 1 dans lequel la base supérieure a une forme rectangulaire, dans lequel le côté distal de la forme rectangulaire se prolonge et devient un triangle pourvu d'arêtes vives.

4. Implant de la revendication 3 dans lequel la deuxième partie (B) est rectangulaire et a la même largeur que la base supérieure.

5. Implant de la revendication 1 dans lequel la troisième partie (C) a une forme rectangulaire avec des coins arrondis.

6. Implant de la revendication 4, dans lequel la première partie (A) a une largeur comprise entre 0,5 et 4 mm.

7. Implant de la revendication 4, dans lequel la deuxième partie (B) a une longueur comprise entre 0,3 et 1,2 mm ; une largeur comprise entre 0,5 et 4 mm ; et une épaisseur comprise entre 0,02 et 0,3 mm.

8. Implant de la revendication 1 dans lequel la troisième partie (C) a une longueur comprise entre 0,5 et 15 mm, une largeur comprise entre 0,7 et 5 mm et une épaisseur comprise entre 0,02 et 0,3 mm.
